# EUROPÄISCHE PATENTSCHRIFT

(11) **EP 0 623 609 B1**
(45) Veröffentlichungstag und Bekanntmachung des Hinweises auf die Patenterteilung: **16.09.1998**
(21) Anmeldenummer: 94106128.5
(22) Anmeldetag: 20.04.1994
(51) Int. Cl.: C07D 319/20, C07C 43/225, C07C 323/03, C07C 41/22, C07C 319/20

(54) **Verfahren zur Herstellung von Perfluoralkoxy- oder Perfluoralkylthiobenzolen**
Process for the preparation of perfluoroalkoxy or perfluoroalkylthiobenzenes
Procédé pour la préparation de perfluoroalkoxy- ou de perfluoroalkylthiobenzenes

(30) Priorität: 03.05.1993 DE 4314498
(43) Veröffentlichungstag der Anmeldung: 09.11.1994
(73) Patentinhaber: BAYER AG, 51368 Leverkusen (DE)
(72) Erfinder: Lui, Norbert, Dr., D-51061 Köln (DE); Marhold, Albrecht, Dr., D-51373 Leverkusen (DE); Gassen, Karl-Rudolf, Dr., D-40882 Ratingen (DE)

(56) Entgegenhaltungen:
- EP-A- 0 100 788
- EP-A- 0 168 344
- EP-A- 0 196 529
- US-A- 5 047 584

## Beschreibung

Die vorliegende Erfindung betrifft ein besonders vorteilhaftes Verfahren zur Herstellung von Perfluoralkoxy-(alkylthio)benzolen aus Perfluorchloralkoxy(alkylthio)-benzolen.

Es ist bekannt, daß man p-α,α-Dichloro-β,β,β-trifluorethoxybenzotrichlorid oder -benzoylchlorid mit Fluorwasserstoff in Gegenwart eines Katalysators in p-Pentafluorethoxy-benzotrifluorid oder -benzoylfluorid überführen kann (US-A 5 047 584). Dabei werden beispielsweise Antimonpentahalogenide als Katalysatoren eingesetzt und in flüssiger Phase bei Normal- oder Überdruck gearbeitet. Die Ausbeuten betragen zwischen 51 und 69 %.

Weiterhin ist bekannt, daß man α,α-Difluorphenylether herstellen kann, indem man die entsprechende α,α-Dichlorverbindungen mit Fluorwasserstoff in Gegenwart einer katalytischen Menge einer Antimon(V)-Verbindung fluoriert (EP-A 168 344). Auch hier wird in flüssiger Phase gearbeitet.

Nachteilig ist, daß dieses Verfahren bei bestimmten Substraten nicht anwendbar ist (siehe Vergleichsbeispiele 1+2) und es nicht kontinuierlich durchführbar ist.

Gemäß der EP-A 196 529 kann man Trifluoranisole herstellen, indem man Trichloranisole in der Gasphase und in Gegenwart von fluoriertem Aluminiumoxid als Katalysator mit Fluorwasserstoff umsetzt.

Es wurde nun ein Verfahren zur Herstellung von Perfluoralkoxy(alkylthio)-benzolen der Formel (I) gefunden 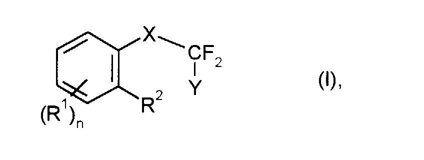 in der
- R¹: für Fluor, Chlor, Brom, C₁-C₄-Fluoralkyl, COF, COCI, C₁-C₄-Alkyl, C₁-C₄-Alkoxy oder C₁-C₄-Fluoralkoxy,
- R²: für Wasserstoff, C₁-C₄-Fluoralkyl, Fluor, Chlor oder Brom und
- Y: für C₁-C₄-Fluoralkyl oder
- R² und Y: gemeinsam für ein Sauerstoff- oder Schwefelatom oder eine -OCHal₂- oder -SCHal₂-Gruppe, wobei dann R² die Position des O- oder S-Atoms markiert und Hal₂ jeweils Cl₂, FCl oder F₂ bedeutet,
- n: für Null, 1 oder 2 und
- X: für Sauerstoff oder Schwefel stehen,
das dadurch gekennzeichnet ist, daß man ein Perfluorchloralkoxy(alkylthio)-benzol der Formel (II) 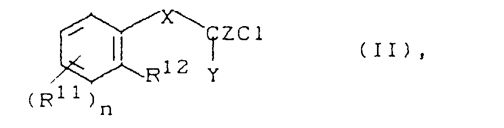 in der
- X, Y und n: die oben angegebene Bedeutung haben und
- R¹¹: für Fluor, Chlor, Brom, C₁-C₄-Halogenalkyl, COF, COCl, C₁-C₄-Alkyl, C₁-C₄-Alkoxy oder C₁-C₄-Halogenalkoxy,
- R¹²: für Wasserstoff, C₁-C₄-Halogenalkyl, Fluor, Chlor oder Brom oder
- R¹² und Y: gemeinsam die oben für R² und Y gemeinsam angegebene Bedeutung haben und
- Z: für Fluor oder Chlor stehen,
mit Fluorwasserstoff in der Gasphase und in Gegenwart von Chloriden, Fluoriden und/oder Oxiden von Kupfer, Chrom, Eisen, Wismut, Zink, Lanthan, Cer, Zirkon, Vanadin, Molybdän, Wolfram und/oder Nickel als Katalysator umsetzt.

Die Ausgangsprodukte für das erfindungsgemäße Verfahren, nämlich Perfluorchloralkoxy(alkylthio)-benzole der Formel (II), sind bekannte Verbindungen (siehe z.B. EP-A 168 344, DE-A 3 329 126 und DE-A 3 409 438) oder auf analoge Weise wie die bekannten zugänglich.

Wenn man Verbindungen der Formel (II) einsetzt, bei denen R¹¹ für einen Halogenalkyl- oder Halogenalkoxyrest und/oder R¹² für einen Halogenalkylrest stehen und wenigstens einer dieser Reste wenigstens ein von Fluor verschiedenes Halogenatom enthält, so werden im erfindungsgemäßen Verfahren solche von Fluor verschiedene Halogenatome gegen Fluoratome ausgetauscht.

Vorzugsweise setzt man in das erfindungsgemäße Verfahren Perfluorchloralkoxybenzole der Formel (II) ein, bei denen
- R¹¹: für Fluor, Chlor, C₁-C₂-Halogenalkyl oder C₁-C₂-Alkoxy,
- R¹²: für Wasserstoff,
- Y: für Trifluormethyl oder
- R¹² und Y: gemeinsam für ein Sauerstoffatom oder eine OCF₂-, OCCl₂- oder OCFCl-Gruppe,
- X: für Sauerstoff und
- n: für Null oder 1 stehen
und erhält vorzugsweise die entsprechenden Perfluoralkoxy-benzole der Formel (I). Besonders bevorzugt setzt man in das erfindungsgemäße Verfahren 2,2,3,3-Tetrachlorbenzodioxan, 2,2-Dichlor-3,3-difluorbenzodioxan, 2-Chlor-2,3,3-trifluorbenzodioxan, 4-(1,1-Dichlor-2,2,2-trifluorethoxy)-chlorbenzol oder 4-(1,1-Dichlor-2,2,2-trifluor-ethylthio)methylbenzol ein und erhält 2,2,3,3-Tetrafluorbenzodioxan, 4-(Perfluorethoxy)-chlorbenzol oder 4-(Perfluorethylthio)-methylbenzol.

Das erfindungsgemäße Verfahren wird in der Gasphase durchgeführt. Das bedeutet, die Perfluorchloralkoxy-(alkylthio)-benzole der Formel (II) werden zusamrnen mit Fluorwasserstoff gasförmig über einen z.B. fest angeordneten oder im Wirbelbett befindlichen Katalysator geführt. Gegebenenfalls kann zusätzlich ein Inertgas zugesetzt werden. Die Reaktionstemperatur kann beispielsweise im Bereich 200 bis 500°C liegen, vorzugsweise bei 250 bis 450°C. Der Druck wird beispielsweise im Bereich von 0,5 bis 3 bar so gewählt, daß die o.a. Reaktanden sich in der Gasphase befinden. Häufig kann bei entsprechend hohen Reaktionstemperaturen bei Normaldruck arbeiten.

Bei dem eingesetzten Fluorwasserstoff kann es sich um die handelsübliche, wasserfreie Qualität handeln.

Bevorzugte Katalysatoren sind Chlor(III)-salze alleine oder im Gemisch mit Chloriden der anderen genannten Metalle und/oder deren Fluoriden und/oder deren Oxiden. Die Katalysatoren können als solche, z.B. in stückiger Form, verwendet werden, aber auch aufgebracht auf einem Trägermaterial, beispielsweise Aluminiumoxid, Magnesiumoxid, Magnesiumfluorid, Calciumfluorid, Zinkchlorid und/oder Graphit.

Im allgemeinen ist es vorteilhaft, den Katalysator, bevor er mit einer Verbindung der Formel (II) beaufschlagt wird, mit Fluorwasserstoff und anschließend elementarem Chlor vorzubehandeln.

Die Menge, in der beim erfindungsgemäßen Verfahren Katalysatoren eingesetzt werden, ist nicht kritisch. Aus wirtschaftlichen Erwägungen wählt man die Katalysatormenge vorteilhafter Weise so, daß ein Umsatz von mindestens 70 % erreicht wird. Beispielsweise kann man pro Stunde 50 g bis 5 kg des Perfluorchloralkoxy-(alkylthio)-benzols der Formel (II) über einen Liter Katalysator leiten.

Fluorwasserstoff kann beispielsweise in Mengen von 0,5 bis 10 Mol, bezogen auf ein auszutauschendes Chlor-Äquivalent, eingesetzt werden. Vorzugsweise setzt man bezogen auf ein auszutauschendes Chlor-Äquivalent 1 bis 8 Mol Fluorwasserstoff ein.

Das nach Passieren des Katalysators vorliegende Reaktionsgemisch kann nach Abkühlung und Kondensation beispielsweise so aufgearbeitet werden, daß man, sofern noch Fluorwasserstoff vorhanden ist, diesen abtrennt, z.B. durch Phasentrennung oder durch Destillation, und den Rückstand fraktioniert destilliert oder den vom Fluorwasserstoff befreiten Rückstand auf Eis gibt, die sich bildende organische Phase abtrennt und diese fraktioniert destilliert.

Das erfindungsgemäße Verfahren zeichnet sich dadurch aus, daß es Perfluor-alkoxy(alkylthio)-benzole der Formel (I) in hohen Ausbeuten und Reinheiten bei langen Katalysatorstandzeiten liefert. Es kann auch mit Vorteil für solche Edukte gebraucht werden, die sich in der Flüssigphase praktisch nicht in gewünschter Weise umsetzen (siehe Vergleichsbeispiele 1+2).

Im Hinblick auf den eingangs geschilderten Stand der Technik war das völlig überraschend.

### Beispiele

### Vergleichsbeispiel 1

50 g 2-Chlor-2,3,3-trifluorbenzodioxan und 25 g Antimontrifluorid wurden auf 180°C erhitzt. Nach 2-stündigem Sieden am Rückfluß wurden 2 ml Antimonpentachlorid hinzugefügt und noch für 3 Stunden bei 150°C gehalten. Nach dem Abkühlen auf Raumtemperatur wurde der Ansatz auf ein Gemisch von 200 ml Wasser und 50 ml konz. Salzsäure gegossen, die organische Phase abgetrennt und destilliert. Es wurden 8 g 2,2,3,3-Tetrafluorobenzodioxan und 22 g unverändertes Ausgangsprodukt erhalten.

### Vergleichsbeispiel 2

500 ml Fluorwasserstoff, 5 ml Antimonpentachlorid und 150 g 2-Chlor-2,3,3 2-Chlor-2,3,3-trifluorobenzodioxan wurden im geschlossenen Gefäß für 6 Stunden auf 140°C erhitzt, danach überschüssiger Fluorwasserstoff abgezogen, der Rückstand in Methylenchlorid aufgenommen, alkalisch gestellt und mit Wasserdampf destilliert. Die Redestillation ergab 27 g 2,2,3,3-Tetrafluorobenzodioxan mit einem Siedepunkt von 142 bis 146°C bei 1020 mbar.

### Vergleichsbeispiel 3

In einem Nickelrohr, das von außen elektrisch beheizbar war und eine lichte Weite von 30 mm aufwies, wurden 350 ml Aluminiumoxid (SPH 501) mit Fluorwasserstoff aktiviert und bei 315°C pro Stunde 150 ml Fluorwasserstoff (gemessen als Flüssigkeit) und 148 g 2-Chlor-2,3,3-trifluorbenzodioxan über den Katalysator geleitet. Nach 2 Stunden wurde aus dem gasförmigen Reaktionsgemisch eine Probe genommen und gaschromatografisch untersucht. Die Probe enthielt nur 3 Gew.-% 2,2,3,3-Tetrafluor-1,3-benzodioxan.

### Erfindungsgemäßes Beispiel 1

600 g Cr(NO₃)₃ x 9H₂O wurden in 300 ml Wasser aufgelöst. Zu dieser Lösung wurden 420 g Magnesiumoxid und 420 g Graphit hinzugefügt und die entstehende pastöse Masse verknetet. Das daraus erhaltene pastöse Produkt wurde zu Würfeln mit einer Kantenlänge von 0,5 cm zerkleinert und dann 16 Stunden bei 100°C getrocknet.

350 ml des so hergestellten Katalysators wurden in das auch im Vergleichsbeispiel 3 verwendete Nickelrohr eingefüllt und dann im Verlauf von 3 Stunden 5 Mol Fluorwasserstoff bei 350°C hindurchgeleitet. Der Fluorwasserstoff wurde im Gemisch mit Stickstoff (Molverhältnis 1:2) eingesetzt. Anschließend wurden für 4 Stunden 35 g/h Chlorgas mit aufgegeben.

Schließlich wurden bei 300°C pro Stunde 170 ml Fluorwasserstoff (gemessen als Flüssigkeit) und 148 g 2-Chlor-2,3,3-trifluorbenzodioxan über den Katalysator geleitet. Im Verlauf von 7 Stunden wurden 930 g 2,2,3,3-Tetrafluorbenzodioxan erhalten. Das entspricht einem Fluorierungsgrad von 97 %.

### Erfindungsgemäßes Beispiel 2

500 g eines aus Tonerde (SPH 501) bestehenden stückigen Trägermaterials wurde mit 125 g CrCl₃ x 6H₂O, gelöst in 800 ml Wasser, getränkt, anschließend getrocknet, mit einer 25 gew.-%igen Ammoniaklösung behandelt, dann salzfrei gewaschen und getrocknet.

In das auch im Vergleichsbeispiel 3 verwendete Nickelrohr wurden 400 ml des so hergestellten Katalysators eingefüllt und anschließend bei 350°C im Verlaufe von 3 Stunden 5 Mol Fluorwasserstoff hindurchgeleitet. Zum Einsatz kam ein Gemisch von Fluorwasserstoff und stickstoff im Molverhältnis 1:2. Danach wurde 4 Stunden lang 35 g/h Chlorgas mit aufgegeben.

Anschließend wurden bei 300°C pro Stunde 140 ml Fluorwasserstoff (gemessen als Flüssigkeit) und 110 g 2-Chlor-2,3,3-trifluorbenzodioxan über den Katalysator geleitet. Im Verlaufe von 6 Stunden wurden 600 g 2,2,3,3-Tetrafluorbenzodioxan erhalten. Das entspricht einer Ausbeute von 98 %.

### Erfindungsgemäßes Beispiel 3

Es wurde verfahren wie im erfindungsgemäßen Beispiel 2, jedoch wurden bei 300°C pro Stunde 110 ml Fluorwasserstoff (gemessen als Flüssigkeit) und 100 g 4-(1,1-Dichlor-2,2,2-trifluorethoxy)-chlorbenzol über den Katalysator geleitet. Im Verlauf von 2 Stunden wurden 165 g 4-(Periluorethoxy)-chlorbenzol erhalten. Das entspricht einer Ausbeute von 93,5 %

### Erfindungsgemäßes Beispiel 4

Es wurde verfahren wie in Beispiel 2, jedoch wurden bei 300°C pro Stunde 110 ml Fluorwasserstoff und 28 g 4-(1,1-Dichlor-2,2,2-trifluorethylthio)-methylbenzol über den Katalysator geleitet. Im Verlaufe von 2 Stunden wurden 47,3 g 4-(Perfluorethylthio)-methylbenzol erhalten. Das entspricht einer Ausbeute von 95 %.

¹H- und ¹⁹F-NMR-Daten, von 4-(Perfluorethylthio)-methylbenzol: ¹H-NMR δ = 2,38 (s, 3H), 7,2 (d, 2H), 7,55 (d,2H); ¹⁹F-NMR δ = -83 (t, 3F), -92,67 (q, 2F).

## Patentansprüche

1. Verfahren zur Herstellung von Perfluoralkoxy(alkylthio)-benzolen der Formel (1) 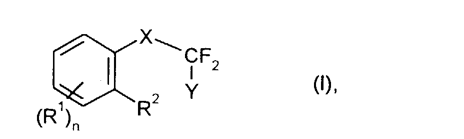 in der
R¹ für Fluor, Chlor, Brom, C₁-C₄-Fluoralkyl, COF, COCl, C₁-C₄-Alkyl, C₁-C₄-Alkoxy oder C₁-C₄-Fluoralkoxy,
R² für Wasserstoff, C₁-C₄-Fluoralkyl, Fluor, Chlor oder Brom und
Y für C₁-C₄-Fluoralkyl oder
R² und Y gemeinsam für ein Sauerstoff- oder Schwefelatom oder eine -OCHal₁- oder -SCHal₂-Gruppe, wobei dann R² die Position des O-oder S-Atoms markiert und Hal₂ jeweils Cl₂, FCl oder F₂ bedeutet,
n für Null, 1 oder 2 und
X für Sauerstoff oder Schwefel stehen,
dadurch gekennzeichnet, daß man ein Perfluorchloralkoxy(alkylthio)-benzol der Formel (II) 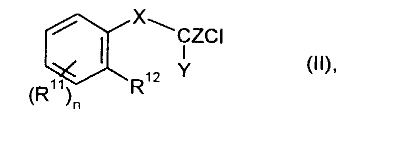 in der
X, Y und n die oben angegebene Bedeutung haben und
R¹¹ für Fluor, Chlor, Brom, C₁-C₄-Halogenalkyl, COF, COCI, C₁-C₄-Alkyl, C₁-C₄-Alkoxy oder C₁-C₄-Halogenalkoxy,
R¹² für Wasserstoff, C₁-C₄-Halogenalkyl, Fluor, Chlor oder Brom oder
R¹² und Y gemeinsam die oben für R² und Y gemeinsam angegebene Bedeutung haben und
Z für Fluor oder Chlor stehen,
mit Fluorwasserstoff in der Gasphase und in Gegenwart von Chloriden, Fluoriden und/oder Oxiden von Kupfer, Chrom, Eisen, Wismut, Zink, Lanthan, Cer, Zirkon, Vanadin, Molybdän, Wolfram und/oder Nickel als Katalysator umsetzt.

2. Verfahren nach Anspruch 1, dadurch gekennzeichnet, daß man Perfluorchloralkoxy-benzole der Formel (II) einsetzt, bei denen
R¹¹ für Fluor, Chlor, C₁-C₂-Halogenalkyl oder C₁-C₂-Alkoxy,
R¹² für Wasserstoff,
Y für Trifluormethyl oder
R¹² und Y gemeinsam für ein Sauersstoffatom oder eine OCF₂-, OCCl₂- oder OCFCI-Gruppe,
X für Sauerstoff und
n für Null oder 1 stehen.

3. Verfahren nach Ansprüchen 1 und 2, dadurch gekennzeichnet, daß man es bei Temperaturen im Bereich 200 bis 500°C und solchen Drucken im Bereich 0,5 bis 3 bar durchführt, daß die Reaktanden sich in der Gasphase befinden.

4. Verfahren nach Ansprüchen 1 bis 3, dadurch gekennzeichnet, daß man Chrom(111)-salze als Katalysatoren einsetzt.

5. Verfahren nach Ansprüchen 1 bis 4, dadurch gekennzeichnet, daß man pro Stunde 50 g bis 5 kg des Perfluorchloralkoxy(alkylthio)-benzols der Formel (II) über einen Katalysator leitet.

6. Verfahren nach Ansprüchen 1 bis 5, dadurch gekennzeichnet, daß man Fluorwasserstoff in Mengen von 0,5 bis 10 Mol, bezogen auf ein auszutauschendes Chlor-Äquivalent, einsetzt.

## Claims

1. Process for preparing perfluoroalkoxy(alkylthio)-benzenes of the formula (I) 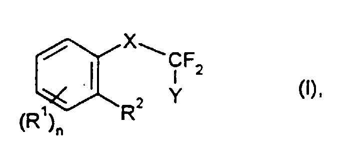 in which
R¹ represents fluorine, chlorine, bromine, C₁-C₄-fluoroalkyl, COF, COCl, C₁-C₄-alkyl, C₁-C₄-alkoxy or C₁-C₄-fluoroalkoxy,
R² represents hydrogen, C₁-C₄-fluoroalkyl, fluorine, chlorine or bromine and
Y represents C₁-C₄-fluoroalkyl or
R² and Y together represent an oxygen or sulphur atom or an -OCHal₂- or -SCHal₂- group, with R² then marking the position of the O or S atom and Hal₂ in each case being Cl₂, FCl or F₂,
n represents zero, 1 or 2 and
X represents oxygen or sulphur,
characterized in that a perfluorochloroalkoxy(alkylthio)-benzene of the formula (II) 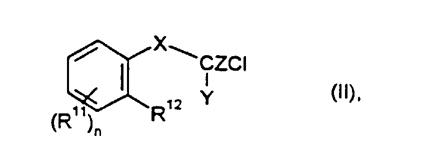 in which
X, Y and n have the meaning given above and
R¹¹ represents fluorine, chlorine, bromine, C₁-C₄-halogenoalkyl, COF, COCl, C₁-C₄-alkyl, C₁-C₄-alkoxy or C₁-C₄-halogenoalkoxy,
R¹² represents hydrogen, C₁-C₄-halogenoalkyl, fluorine, chlorine or bromine or
R¹² and Y together have the meaning given above for R² and Y together and
Z represents fluorine or chlorine,
is reacted with hydrogen fluoride in the gas phase and in the presence of chlorides, fluorides and/or oxides of copper, chromium, iron, bismuth, zinc, lanthanum, cerium, zirconium, vanadium, molybdenum, tungsten and/or nickel as catalyst.

2. Process according to Claim 1, characterized in that the perfluorochloroalkoxy-benzenes of the formula (II) which are used are ones in which
R¹¹ represents fluorine, chlorine, C₁-C₂-halogenoalkyl or C₁-C₂-alkoxy,
R¹² represents hydrogen,
Y represents trifluoromethyl or
R¹² and Y together represent an oxygen atom or an OCF₂-, OCCl₂- or OCFCl- group,
X represents oxygen and
n represents zero or 1.

3. Process according to Claims 1 and 2, characterized in that it is carried out at temperatures in the range from 200 to 500°C and at such pressures in the range from 0.5 to 3 bar that the reactants are in the gas phase.

4. Process according to Claims 1 to 3, characterized in that the catalysts used are chromium(III) salts.

5. Process according to Claims 1 to 4, characterized in that from 50 g to 5 kg per hour of the perfluorochloroalkoxy(alkylthio) -benzene of the formula (II) are passed over a catalyst.

6. Process according to Claims 1 to 5, characterized in that hydrogen fluoride is used in amounts from 0.5 to 10 mol, based on one equivalent of chlorine to be replaced.

## Revendications

1. Procédé pour préparer les perfluoralcoxy(alkylthio)benzènes de formule (I) 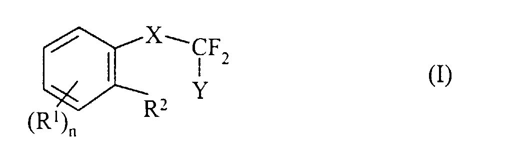 dans laquelle
R¹ représente le fluor, le chlore, le brome, un groupe fluoralkyle en C₁-C₄, COF, COCl, alkyle en C₁-C₄, alcoxy en C₁-C₄ ou fluoralcoxy en C₁-C₄,
R² représente l'hydrogène, un groupe fluoralkyle en C₁-C₄, le fluor, le chlore ou le brome et
Y représente un groupe fluoralkyle en C₁-C₄, ou bien
R² et Y représentent ensemble un atome d'oxygène ou de soufre ou un groupe -OCHal₂ ou -SCHal₂, R² marquant alors la position de l'atome d'oxygène ou de soufre et Hal₂ représentant Cl₂, FCl ou F₂,
n est égal à 0, 1 ou 2 et
X représente l'oxygène ou le soufre,
caractérisé en ce que l'on fait réagir un perfluorochloralcoxy-(alkylthio)benzène de formule (II) 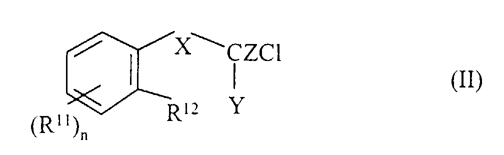 dans laquelle
X, Y et n ont les significations indiquées ci-dessus et
R¹¹ représente le fluor, le chlore, le brome, un groupe halogénoalkyle en C₁-C₄, COF, COCl, alkyle en C₁-C₄, alcoxy en C₁-C₄ ou halogénoalcoxy en C₁-C₄,
R¹² représente l'hydrogène, un groupe halogénoalkyle en C₁-C₄, le fluor, le chlore ou le brome, ou bien
R¹² et Y ensemble, ont les significations indiquées ci-dessus dans ce cas et
Z représente le fluor ou le chlore,
avec le fluorure d'hydrogène en phase gazeuse et en présence de catalyseurs consistant en chlorures, fluorures et/ou oxydes du cuivre, du chrome, du fer, du bismuth, du zinc, du lanthane, du cérium, du zirconium, du vanadium, du molybdène, du tungstène et/ou du nickel.

2. Procédé selon la revendication 1, caractérisé en ce que l'on met en oeuvre des perfluorochloralcoxybenzènes de formule (II) dans laquelle
R¹¹ représente le fluor, le chlore, un groupe halogénoalkyle en C₁-C₂ ou alcoxy en C₁-C₂,
R¹² représente l'hydrogène,
Y représente un groupe trifluorométhyle, ou bien
R¹² et Y représentent ensemble un atome d'oxygène ou un groupe -OCF₂, -OCCl₂ ou -OCFCl,
X représente l'oxygène, et
n est égal à 0 ou 1.

3. Procédé selon les revendications 1 et 2, caractérisé en ce que l'on opère à des températures dans l'intervalle de 200 à 500°C et des pressions dans l'intervalle de 0,5 à 3 bar, en sorte que les réactifs soient en phase gazeuse.

4. Procédé selon les revendications 1 à 3, caractérisé en ce que l'on utilise en tant que catalyseurs des sels de chrome-(III).

5. Procédé selon les revendications 1 à 4, caractérisé en ce que l'on envoie sur un catalyseur, à l'heure, 50g à 5 kg du perfluorochloralcoxy-(alkylthio)benzène de formule (II).

6. Procédé selon les revendications 1 à 5, caractérisé en ce que l'on met en oeuvre le fluorure d'hydrogène en quantité de 0,5 à 10 mol par équivalent de chlore à échanger.
